(19) Europäisches Patentamt
European Patent Office
Office européen des brevets

(11) EP 4 098 285 A1

(12) **EUROPEAN PATENT APPLICATION**
published in accordance with Art. 153(4) EPC

(43) Date of publication:
**07.12.2022 Bulletin 2022/49**

(21) Application number: **21748464.1**

(22) Date of filing: **25.01.2021**

(51) International Patent Classification (IPC):
***A61L 27/18*** *(2006.01)* ***A61L 27/52*** *(2006.01)*
***A61L 27/54*** *(2006.01)* ***A61L 27/56*** *(2006.01)*
***A61L 27/60*** *(2006.01)*

(52) Cooperative Patent Classification (CPC):
**A61L 27/18; A61L 27/52; A61L 27/54; A61L 27/56; A61L 27/60**

(86) International application number:
**PCT/JP2021/002405**

(87) International publication number:
**WO 2021/153489 (05.08.2021 Gazette 2021/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.01.2020 JP 2020011428**

(71) Applicants:
- **The University of Tokyo**
  **Bunkyo-ku, Tokyo 113-8654 (JP)**
- **Gellycle Co., Ltd.**
  **Tokyo 113-0033 (JP)**

(72) Inventors:
- **SAKAI Takamasa**
  **Tokyo 113-8654 (JP)**
- **HOJO Hironori**
  **Tokyo 113-8654 (JP)**
- **TANI Shoichiro**
  **Tokyo 113-8654 (JP)**
- **MASUI Kosuke**
  **Tokyo 113-0033 (JP)**
- **NARITA Shinichi**
  **Tokyo 113-0033 (JP)**

(74) Representative: **Symbiosis IP Limited**
**The Innovation Centre**
**217 Portobello**
**Sheffield, South Yorkshire S1 4DP (GB)**

(54) **GEL MATERIAL FOR REGENERATIVE MEDICINE**

(57) An object of the present invention is to provide a novel use in which a polymer gel material having a μm-scale porous structure can be suitably used.

A gel material for regenerative medicine comprising a polymer gel in which hydrophilic polymer units are crosslinked with each other, wherein the polymer gel contains water as a solvent and has a three-dimensional network structure having two regions: a first region in which the polymer units are densely present and a second region in which the polymer units are sparsely present, and a mesh size composed of the first region is the range of 1 to 500 μm.

[Fig. 5]

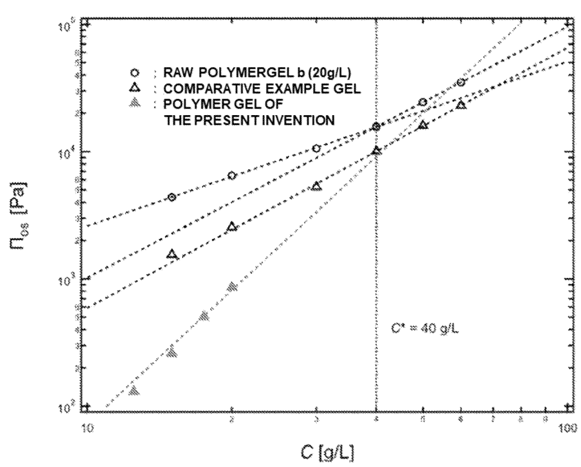


EP 4 098 285 A1

## Description

### Technical Field

The present invention relates to a gel material for regenerative medicine which contains a polymer gel including two regions: a dense phase and a dilute phase of a hydrophilic polymer component and having a porous structure, and a kit for producing the gel material.

### Background Art

In recent years, polymer gels having a network-like structure have characteristics such as excellent water retention ability and biocompatibility, and therefore the polymer gels are materials that are expected to be applied to various applications such as medical applications (e.g., artificial tissues, scaffold materials for regeneration, sealing, adhesion prevention, drug delivery, and contact lenses), and applications for sensors and surface coatings (for example, Non Patent Literature 1). In particular, in these applications, it is desired to develop a polymer material having a μm-scale porous structure.

However, conventionally, in order to obtain the μm-scale porous structure, it has been necessary that a polymer material is produced using a top-down method such as microfabrication of a gel or polymer structure produced in advance by lithography or the like, or a solvent-insoluble polymer material. On the other hand, when a solvophilic polymer raw material is used, the material itself is dissolved in the solvent in the first place, or only a gel having a small (nm-scale) porous structure can be produced. In addition, an example in which a gel material having such a porous structure is applied to the regeneration and repair of biological tissues has not been reported so far.

### Citation List

### Non Patent Literature

Non Patent Literature 1: Sakai et al., Macromolecules, 41, 5379-5384, 2008

### Summary of Invention

### Technical Problem

Therefore, an object of the present invention is to provide a gel material with a μm-scale porous structure formed by solvophilic polymers, and further to provide a novel use in which such a gel material can be suitably used.

### Solution to Problem

As a result of intensive studies to solve the above problems, the present inventors have found that a polymer gel obtained by crosslinking hydrophilic polymer raw materials under specific conditions behaves like a phase separation in poorly soluble polymers, and has a peculiar structure which forms a sponge-like three-dimensional network structure (μm-scale porous structure) including a dense region in which polymer components are densely present and a dilute region in which polymer components are sparsely present, and the structure has not been found in conventional polymer gels. Further, they have found that, in regeneration and repair of biological tissues such as bone, a gel material containing the polymer gel is applied to an affected part, thereby allowing for promotion of the regeneration and repair of biological tissues. Based on these findings, they have completed the present invention.

Specifically, according to the present invention of a first aspect, there is provided

<1> a gel material for regenerative medicine including a polymer gel in which hydrophilic polymer units are crosslinked with each other, wherein the polymer gel contains water as a solvent and has a three-dimensional network structure having two regions: a first region in which the polymer units are densely present and a second region in which the polymer units are sparsely present, and a mesh size composed of the first region is the range of 1 to 500 μm.

Further, according to preferred aspects of the gel material for regenerative medicine of the present invention, there are provided:

<2> the gel material for regenerative medicine according to <1> above, wherein the gel material has a lower transmittance than a transmittance of the polymer units before gelation;

<3> the gel material for regenerative medicine according to <1> or <2> above, wherein the gel material has an osmotic pressure of 1/5 to 1/2 of an osmotic pressure of the polymer units before gelation;

<4> the gel material for regenerative medicine according to any one of <1> to <3> above, wherein an osmotic pressure ($\Pi_{os}$) and an elastic pressure ($\Pi_{el}$) after a lapse of a certain period of time from gelation have a relationship of $\Pi_{el} > \Pi_{os}$ ;
<5> the gel material for regenerative medicine according to any one of <1> to <4> above, wherein a polymer concentration in the first region is from 10 to 99 wt%, and a polymer concentration in the second region is from 0 to 1 wt%;
<6> the gel material for regenerative medicine according to any one of <1> to <5> above, wherein the polymer gel has a polymer content of 5 wt% or less;
<7> the gel material for regenerative medicine according to any one of <1> to <6> above, wherein the polymer units are polymers having a polyethylene glycol backbone or a polyvinyl backbone;
<8> the gel material for regenerative medicine according to any one of <1> to <7> above, wherein the polymer units include a first polymer unit having one or more nucleophilic functional groups in a side chain or at an end and a second polymer unit having one or more electrophilic functional groups in a side chain or at an end;
<9> the gel material for regenerative medicine according to <8> above, wherein the nucleophilic functional groups are selected from the group consisting of a thiol group and an amino group, and the electrophilic functional groups are selected from the group consisting of a maleimidyl group, an N-hydroxy-succinimidyl (NHS) group, a sulfosuccinimidyl group, a phthalimidyl group, an imidazolyl group, an acryloyl group, a nitrophenyl group, and $-CO_2PhNO_2$;
<10> the gel material for regenerative medicine according to any one of <1> to <9> above, which is used for regeneration of a tissue;
<11> the gel material for regenerative medicine according to <10> above, wherein the tissue is bone, cartilage, skin, or nerve; and
<12> a treatment process using the gel material for regenerative medicine according to any one of <1> to <11> above.

In another aspect, the present invention relates to a kit for producing a gel material for regenerative medicine, and provides:

<13> a kit for producing a gel material for regenerative medicine, the kit including the following two types of solutions (A) and (B) stored without being mixed with each other: (A) an aqueous solution containing a hydrophilic first raw polymer having a concentration of less than an overlapping concentration and equal to or more than a critical gelation concentration; and (B) an aqueous solution containing a hydrophilic second raw polymer having a concentration of less than an overlapping concentration and equal to or more than a critical gelation concentration; wherein the solution (A) and/or the solution (B)further contains a non-reactive polymer having no functional group capable of crosslinking with the raw polymer in the molecule, a polymer gel obtained by mixing the solutions (A) and (B) is a polymer gel in which the first raw polymer and the second raw polymer are crosslinked with each other, and the polymer gel has a structure including two regions: a first region in which polymer units derived from the raw polymers are densely present and a second region in which polymer units derived from the raw polymers are sparsely present; and
<14> a kit for producing a gel material for regenerative medicine, the kit including the following two types of solutions (A') and (B') stored without being mixed with each other: (A') an aqueous solution containing a first gel precursor, wherein the first gel precursor is obtained by crosslinking hydrophilic raw polymers under conditions of less than an overlapping concentration and less than a critical gelation concentration, and the first gel precursor has a relationship of G' < G" between a storage modulus G' and a loss modulus G"; and (B') an aqueous solution containing a second gel precursor, wherein the second gel precursor is obtained by crosslinking hydrophilic raw polymers under conditions of less than an overlapping concentration and less than a critical gelation concentration, and the second gel precursor has a relationship of G' < G" between a storage modulus G' and a loss modulus G"; wherein the solution (A') and/or the solution (B')further contains a crosslinking agent, a polymer gel obtained by mixing the solutions (A') and (B') is a polymer gel in which the first raw polymer and the second raw polymer are crosslinked with each other, and the polymer gel has a structure including two regions: a first region in which polymer units derived from the raw polymers are densely present and a second region in which polymer units derived from the raw polymers are sparsely present.

According to preferred aspects of the kit of the present invention, there are provided:

<15> the kit according to <13> or <14> above, wherein the raw polymers are polymers having a polyethylene glycol backbone or a polyvinyl backbone;
<16> the kit according to any one of <13> to <15> above, wherein the raw polymers include a first polymer having one or more nucleophilic functional groups in a side chain or at an end and a second polymer having one or more electrophilic functional groups in a side chain or at an end;
<17> The kit according to <16> above, wherein the nucleophilic functional groups are selected from the group

consisting of a thiol group and an amino group, and the electrophilic functional groups are selected from the group consisting of a maleimidyl group, an N-hydroxy-succinimidyl (NHS) group, a sulfosuccinimidyl group, a phthalimidyl group, an imidazolyl group, an acryloyl group, a nitrophenyl group, and $-CO_2PhNO_2$;
<18> the kit according to <13> above, wherein the non-reactive polymer is polyethylene glycol or cellulose, which has no crosslinking reactive group;
<19> the kit according to <14> above, wherein the first gel precursor and the second gel precursor both have a first polymer having one or more nucleophilic functional groups in a side chain or at an end and a second polymer having one or more electrophilic functional groups in a side chain or at an end, the first gel precursor has a higher first polymer content than a second polymer content, and the second gel precursor has a higher second polymer content than a first polymer content;
<20> the kit according to <14> above, wherein the gel precursors have a diameter of 10 to 1000 nm;
<21> a process for producing a gel material for regenerative medicine in vivo using the kit according to any one of <13> to <20> above; and
<22> use of the kit according to any one of <13> to <20> above in tissue regeneration treatment.

Advantageous Effects of Invention

In regeneration and repair of biological tissues, it is possible to promote the regeneration and repair of biological tissues such as bone by applying the gel material for regenerative medicine of the present invention to an affected part. Specifically, as shown in Examples, a bone defect is filled with the gel material for regenerative medicine of the present invention, so that it is possible to obtain an effect of improving bone union and growth of new bone in the bone defect.
In addition, since it is possible to form the gel material for regenerative medicine in situ in the living body by using the kit of the present invention, the gel material for regenerative medicine can be used as a gel material that can be injected into a closed or semi-closed cavity in the living body.

Brief Description of Drawings

Fig. 1 is a graph showing a time scale of a gelation reaction in a polymer gel of the present invention produced in Example 2.
Fig. 2 is a graph showing changes in transmittance in a gelation step of the present invention.
Fig. 3 is a graph showing changes in transmittance of a comparative example gel which does not pass through a gel precursor stage.
Fig. 4 is a graph showing temporal changes in the degree of swelling of the polymer gel of the present invention.
Fig. 5 is a graph showing changes in osmotic pressure ($\Pi_{os}$) of the polymer gel of the present invention.
Fig. 6 shows fluorescence microscope images of the polymer gel of the present invention (right) and the comparative example gel (left).
Fig. 7A shows micro-CT images of polymer gel-unfilled rat tibial sites (a to c) and polymer gel-filled rat tibial sites (d to f) 2, 3, and 4 weeks after bone defect formation. Fig. 7B shows HE-stained images of polymer gel-unfilled rat tibial sites (g and h) and polymer gel-filled rat tibial sites (i and j) 4 weeks after bone defect formation (each black bar: 5 mm; enlarged views of broken-lined squares in images of g and h are i and j, respectively).
Fig. 8 shows micro-CT images of a sham group (NTC; negative control), a group filled with the comparative example gel ("Tetra-PEG gel"), and a group filled with the polymer gel of the present invention ("Tetra-PEG sponge") 2, 4, and 8 weeks after bone defect formation.
Fig. 9 shows images after subcutaneous implantation of the polymer gel of the present invention ("Oligo-TetraPEG gel") and the comparative example gel (Tetra-PEG gel) in a rat.

Description of Embodiments

Hereinafter, embodiments of the present invention will be described. The scope of the present invention is not restricted to these explanations, and various modifications other than the following examples can be made without departing the spirit of the present invention.

1. Gel Material for Regenerative Medicine of Present Invention

The gel material for regenerative medicine of the present invention includes a polymer gel having a sponge-like porous structure of a $\mu$m scale.

In the present specification, the term "regenerative medicine" means a treatment for regenerating or repairing biological tissues in which deficiency, damage, or functional deterioration is caused by nature or a factor such as disease, injury, accident or aging, and restoring the function. Therefore, the present invention is not limited to the treatment for transplanting autologous somatic stem cells, somatic stem cells from another person, pluripotent stem cells such as iPS cells, or tissues obtained from these cells ("regenerative medicine in a narrow sense").
In the present specification, the "tissue" to be a target of regenerative medicine is not particularly limited as long as it is a biological tissue requiring regeneration or repair, and examples thereof include bone, cartilage, skin, nerve, organ, and the like.
Hereinafter, a polymer gel as a main component of the gel material for regenerative medicine and a process for producing the polymer gel will be described in detail.

(1) Polymer Gel

The polymer gel contained in the gel material for regenerative medicine of the present invention is a polymer gel gelled by crosslinked hydrophilic polymer units,

wherein the polymer gel is a so-called hydrogel containing water as a solvent, and i) has a three-dimensional network structure having two regions: a first region in which the polymer units are densely present and a second region in which the polymer units are sparsely present, and ii) a mesh size composed of the first region is the range of 1 to 500 μm.

In other words, although the polymer gel in the present invention is formed from the solvophilic polymer units, the polymer gel behaves as if poorly soluble polymers are phase-separated in a solvent, and has a structure in which two regions with different polymer concentrations: a dense phase (first region) in which polymer components are densely present and a dilute phase (second region) in which polymer components are sparsely present, are formed in the gel. The polymer gel forms a sponge-like three-dimensional network structure/porous structure by this phase separation (hereinafter, such a structure may be referred to as "sponge-like porous structure"). The mesh size is also characterized by being on the order of μm, which is much larger than the order of nm obtained by conventional gels. Here, the first region is referred to as "dense phase" in a relative sense that the concentration (density) of the polymer units present in the region is higher than the density in the second region. Preferably, the first region has a concentration (density) of about 100 times that of the second region.
In the present specification, the "gel" is generally a dispersion system of a polymer that has high viscosity and lost fluidity, and refers to a state having a relationship of $G' \geq G''$ between a storage modulus $G'$ and a loss modulus $G''$.
As described above, the polymer gel in the present invention includes a porous structure on the order of μm. Specifically, the mesh size composed of the first region can be as large as 1 to 500 μm, and is preferably from 10 to 100 μm. The mesh size means a length of a long side in a mesh unit (i.e., a pore) in which the outer circumference is formed by the first region as the dense phase. Alternatively, when the mesh unit is substantially circular, it can be the length of its diameter. There is a second region, which is a dilute phase, and/or a solvent in the mesh unit.
Typically, the first region as the dense phase has a polymer concentration of 10 to 99 wt%, and the second region as the dilute phase has a polymer concentration of 0 to 1 wt%, based on the entire gel containing the solvent. Preferably, the first region has a polymer concentration of 40 to 80 wt% and the second region has a polymer concentration of 0.01 to 0.1 wt%.
Further, the polymer content of the entire polymer gel in the present invention is 5 wt% or less, preferably 4 wt% or less, and more preferably 1.5 to 3.0 wt%.
As described above, water is used as the solvent contained in the polymer gel in the present invention. In this case, the polymer gel containing water becomes a hydrogel.
However, in some cases, an alcohol such as ethanol and an organic solvent such as DMSO can be further included.
Hereinafter, the polymer units constituting the polymer gel used in the gel material for regenerative medicine of the present invention and the characteristic physical properties exhibited by the polymer gel will be described.

1-a. Polymer Unit

The polymer units used to form the polymer gel in the present invention are polymers that are solvophilic, i.e., soluble in a solvent contained in the gel. For example, in a case where the gel is a hydrogel containing water as a solvent, the polymer units are hydrophilic polymers. The polymer units can be known polymer units used in the technical field in accordance with the application, shape, and the like of the final gel as long as they are capable of forming a gel by a gelation reaction (such as a crosslinking reaction) in a solution. More specifically, polymer units capable of forming a

network structure, particularly a three-dimensional network structure, by crosslinking of the polymer units with each other in the final gel are preferable.

The hydrophilic polymers used as the polymer units may be preferably polymers having a polyethylene glycol backbone or a polyvinyl backbone. Typical examples of the polymers having a polyethylene glycol backbone include polymers having a plurality of arms of polyethylene glycol backbones, and polymers having four arms of polyethylene glycol backbones; polymers having four arms of polyethylene glycol backbones are particularly preferable. Such a gel including a tetra-branched polyethylene glycol backbone is known as a Tetra-PEG gel, and a network-structure network is constructed by an AB-type cross-end coupling reaction between two types of four-armed polymers having an electrophilic functional group such as an active ester structure and a nucleophilic functional group such as an amino group at each end (Matsunaga et al., Macromolecules, Vol. 42, No. 4, pp. 1344-1351, 2009). In addition, Tetra-PEG gels can also be prepared on site easily by simple two-liquid mixing of each polymer solution, and the gelation time can also be controlled by adjusting the pH and ionic strength during gel preparation. Then, these gels have excellent biocompatibility since the main component is PEG.

Note that polymers having other than a polyethylene glycol backbone can also be used as long as they can be gelled by crosslinking with each other. For example, polymers having a polyvinyl backbone such as methyl methacrylate can also be used.

Although not necessarily limited thereto, a crosslinking unit for reacting two types of polymers: a first polymer unit having one or more nucleophilic functional groups in a side chain or at an end and a second polymer unit having one or more electrophilic functional groups in a side chain or at an end, as the polymer units, is suitable for forming a sponge-like porous structure in the final gel. Here, the total of the nucleophilic functional groups and the electrophilic functional groups is preferably 5 or higher. These functional groups are also preferably present at the ends. Further, the gel precursor can have a composition in which the content of the first polymer units is greater than the content of the second polymer units, or a composition in which the content of the second polymer units is greater than the content of the first polymer units. As described below, in a preferred aspect, a polymer gel can be obtained by once forming two or more types of gel precursors of such different compositions, and crosslinking the gel precursors.

Examples of nucleophilic functional groups present in the polymer units include a thiol group (-SH), an amino group, and the like, and those skilled in the art can appropriately use known nucleophilic functional groups. Preferably, the nucleophilic functional groups are -SH groups. The nucleophilic functional groups may be identical or different, and the nucleophilic functional groups are preferably identical. Having the functional groups be identical makes the reactivity with the electrophilic functional groups that serve to form the crosslinking bonds uniform and makes it easy to obtain a gel having a uniform three-dimensional structure.

Active ester groups can be used as the electrophilic functional groups present in the polymer units. Examples of such active ester groups include a maleimidyl group, an N-hydroxy-succinimidyl (NHS) group, a sulfosuccinimidyl group, a phthalimidyl group, an imidazolyl group, an acryloyl group, a nitrophenyl group, and $-CO_2PhNO_2$ (Ph represents an o-, m-, or p-phenylene group.), and the like. Those skilled in the art can appropriately use known active ester groups. Preferably, the electrophilic functional groups are maleimidyl groups. The electrophilic functional groups may be identical or different, and the electrophilic functional groups are preferably identical. Having the functional groups be identical makes the reactivity with the nucleophilic functional groups that serve to form the crosslinking bonds uniform and makes it easy to obtain a gel having a uniform three-dimensional structure.

Nonlimiting specific examples preferred as polymer units having nucleophilic functional groups at the ends include compounds represented by the following Formula (I) having four polyethylene glycol backbone arms and thiol groups at the ends:

[Chem. 1]

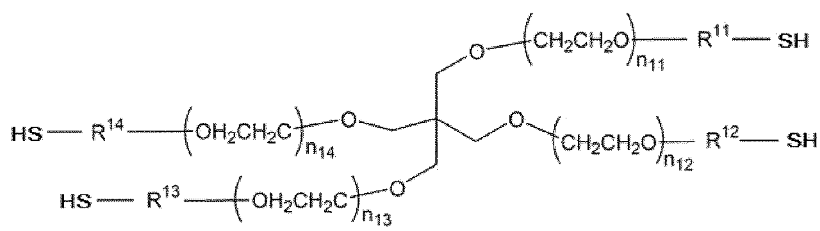

(I)

$n_{11}$ to $n_{14}$ may be identical or different. The closer the values of $n_{11}$ to $n_{14}$ are, the more uniform the three-dimensional structure can be, and the higher the strength becomes. Thus, in order to obtain a high-strength gel, it is preferable that the values are identical. When the values of $n_{11}$ to $n_{14}$ are too high, the strength of the gel is weakened. When the values of $n_{11}$ to $n_{14}$ are too low, the gel is not likely to be formed due to the steric hindrance of the compound.

Therefore, $n_{11}$ to $n_{14}$ are, for example, integer values from 25 to 250, preferably integer values from 35 to 180, more preferably integer values from 50 to 115, and particularly preferably integer values from 50 to 60. The molecular weight is, for example, from $5 \times 10^3$ to $5 \times 10^4$ Da, preferably from $7.5 \times 10^3$ to $3 \times 10^4$ Da, and more preferably from $1 \times 10^4$ to $2 \times 10^4$ Da.

In Formula (I) above, $R^{11}$ to $R^{14}$ are linker sites that connect the functional group and the core portion. $R^{11}$ to $R^{14}$ may be identical or different, and are preferably identical in order to produce a high-strength gel having a uniform three-dimensional structure. $R^{11}$ to $R^{14}$ represent a $C_1$-$C_7$ alkylene group, a $C_2$-$C_7$ alkenylene group, -NH-$R^{15}$-, - CO-$R^{15}$-, -$R^{16}$-O-$R^{17}$-, -$R^{16}$-NH-$R^{17}$-, -$R^{16}$-$CO_2$-$R^{17}$-, -$R^{16}$-$CO_2$-NH-$R^{17}$-, -$R^{16}$-CO-$R^{17}$-, $R^{16}$-NH-CO-$R^{17}$-, or -$R^{16}$-CO-NH-$R^{17}$-. Here, $R^{15}$ represents a $C_1$-$C_7$ alkylene group. $R^{16}$ represents a $C_1$-$C_3$ alkylene group. $R^{17}$ represents a $C_1$-$C_5$ alkylene group.

Here, a "$C_1$-$C_7$ alkylene group" means an optionally branched alkylene group having 1 or more and 7 or less carbon atoms, and means a linear $C_1$-$C_7$ alkylene group or a $C_2$-$C_7$ alkylene group having one or more branches (the number of carbon atoms, including branches, is 2 or more and 7 or less). Examples of $C_1$-$C_7$ alkylene groups include a methylene group, an ethylene group, a propylene group, and a butylene group. Examples of $C_1$-$C_7$ alkylene groups include $-CH_2-$, $-(CH_2)_2-$, $-(CH_2)_3-$, $-CH(CH_3)-$, $-(CH_2)_3-$, $-(CH(CH_3))_2-$, $-(CH_2)_2-CH(CH_3)-$, $-(CH_2)_3-CH(CH_3)-$, $-(CH_2)_2-CH(C_2H_5)-$, - $(CH_2)_6-$, $-(CH_2)_2-C(C_2H_5)_2-$, and $-(CH_2)_3C(CH_3)_2CH_2-$, and the like.

A "$C_2$-$C_7$ alkenylene group" is a linear or branched, alkenylene group having 2 to 7 carbon atoms having one or more double bonds in the chain. Examples include divalent groups having double bonds formed by eliminating 2 to 5 hydrogen atoms of adjacent carbon atoms from the alkylene group.

Meanwhile, nonlimiting specific examples preferred as polymer units having electrophilic functional groups at the ends include compounds represented by the following Formula (II) having four polyethylene glycol backbone arms and maleimidyl groups at the ends:

[Chem. 2]

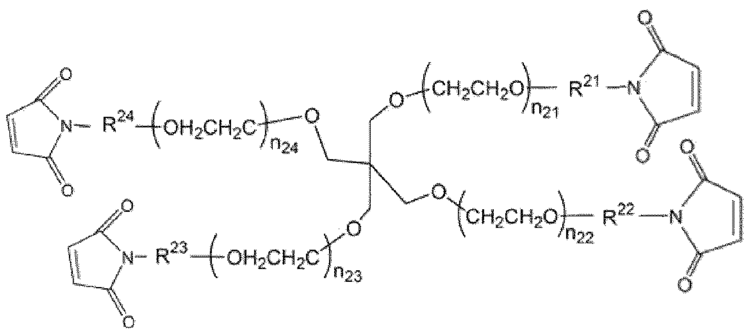

(II)

In Formula (II) above, $n_{21}$ to $n_{24}$ may be identical or different. The closer the values of $n_{21}$ to $n_{24}$ are, the more uniform the three-dimensional structure of the gel can be, and the higher the strength becomes, which is preferable. Thus, the values are preferably identical. When the values of $n_{21}$ to $n_{24}$ are too high, the strength of the gel is weakened. When the values of $n_{21}$ to $n_{24}$ are too low, the gel is not likely to be formed due to the steric hindrance of the compound. Therefore, $n_{21}$ to $n_{24}$ are, for example, integer values from 5 to 300, preferably integer values from 20 to 250, more preferably integer values from 30 to 180, still more preferably integer values from 45 to 115, and yet still more preferably integer values from 45 to 55. The molecular weight of the second tetra-branched compound of the present invention is, for example, from $5 \times 10^3$ to $5 \times 10^4$ Da, preferably from $7.5 \times 10^3$ to $3 \times 10^4$ Da, and more preferably from $1 \times 10^4$ to $2 \times 10^4$ Da.

In Formula (II) above, $R^{21}$ to $R^{24}$ are linker sites that connect the functional group and the core portion. $R^{21}$ to $R^{24}$ may be identical or different, and are preferably identical in order to produce a high-strength gel having a uniform three-dimensional structure. In Formula (II), $R^{21}$ to $R^{24}$ are identical or different and represent a $C_1$-$C_7$ alkylene group, a $C_2$-$C_7$ alkenylene group, -NH-$R^{25}$-, -CO-$R^{25}$-, -$R^{26}$-NH-$R^{27}$-, -$R^{26}$-$CO_2$-$R^{27}$-, -$R^{26}$-$CO_2$-NH-$R^{27}$-, -$R^{26}$-CO-$R^{27}$-, -$R^{26}$-NH-CO-$R^{27}$-, or -$R^{26}$-CO-NH-$R^{27}$-. Here, $R^{25}$ represents a $C_1$-$C_7$ alkylene group. $R^{26}$ represents a $C_1$-$C_3$ alkylene group. $R^{27}$ represents a $C_1$-$C_5$ alkylene group.

In the present specification, the alkylene group and the alkenylene group may have one or more optional substituents. Examples of the substituents include, but are not limited to, an alkoxy group, a halogen atom (which may be any of a fluorine atom, a chlorine atom, a bromine atom, or an iodine atom), amino groups, mono- or disubstituted amino groups, substituted silyl groups, acyl groups, aryl groups, and the like. When an alkyl group has two or more substituents, they may be identical or different. The same is also true of alkyl moieties of other substituents (for example,

alkyloxy groups and aralkyl groups) including alkyl moieties.

Further, in the present specification, when certain functional groups are defined as "optionally substituted," the types of substituents, positions substituted, and number of substituents are not particularly limited, and when they have two or more substituents, the substituents may be identical or different. Examples of substituents include, but are not limited to, alkyl groups, alkoxy groups, hydroxyl groups, carboxyl groups, halogen atoms, sulfo groups, amino groups, alkoxycarbonyl groups, oxo groups, and the like. Other substituents may also be present in these substituents.

1-b. Physical Properties of Polymer Gel

As described above, the polymer gel used in the gel material for regenerative medicine of the present invention has a sponge-like porous structure on the order of μm, formed by the dense phase (first region) and the dilute phase (second region) in the gel, and has characteristic properties in terms of various physical properties due to the structure.

The polymer gel in the present invention has a lower transmittance than a transmittance of the polymer units before gelation. This is because the polymer gel has two regions: the dense phase (first region) and the dilute phase (second region), which have different polymer concentrations, in the gel, and the polymer gel behaves as if poorly soluble polymers are phase-separated in a solvent, and thus the polymer gel is not completely transparent and becomes cloudy. Preferably, the polymer gel has a transmittance of 90 to 96%. In terms of such transmittance, the polymer gel exhibits properties completely different from those of normal polymer gels, which are almost transparent.

The polymer gel in the present invention has an osmotic pressure of 1/5 to 1/2 of an osmotic pressure of the polymer units before gelation. Further, the polymer gel of the present invention has a lower osmotic pressure than a single-phase polymer gel formed from the same polymer units.

In addition, in the polymer gel in the present invention, an osmotic pressure ($\Pi_{os}$) and an elastic pressure ($\Pi_{el}$) after a lapse of a certain period of time from gelation have a relationship of $\Pi_{el} > \Pi_{os}$. The relationship that $\Pi_{el}$ is greater than $\Pi_{os}$ indicates that the gel is in a shrunk state. In contrast, normal polymer gels generally have a relationship of $\Pi_{el} < \Pi_{os}$ and tend to swell.

Although not necessarily restricted by the theory, it is understood that the polymer gel used in the gel material for regenerative medicine of the present invention has a structure such as a two-phase separation, like a dense phase and a dilute phase, and thus the osmotic pressure ($\Pi_{os}$) of the dilute phase is lower than that of a normal single-phase polymer gel, whereas the polymer gel tends to shrink due to an increase in elastic pressure. In these respects, it can be said that the polymer gel in the present invention has characteristic properties that are significantly different from those of conventional polymer gels.

Further, as described above, since the polymer densities of the polymer units are different between the dense phase and the dilute phase, the polymer gel in the present invention can have different water contents in these two regions. Specifically, in the polymer gel in the present invention, the water content of the first region (dense phase) is in a range of 10 to 99%, and the water content of the second region (dilute phase) is in a range of 99 to 100%.

The polymer gel in the present invention can be processed into a variety of shapes such as a thin film in accordance with the application. Any methods known in this technical field can be used for such processing. For example, in the case of a thin film, the thin film can be obtained by a method applying the gel to a flat substrate such as glass in a state of having fluidity prior to complete solidification.

(2) Process for Producing Polymer Gel

Next, the process for producing a polymer gel used in the gel material for regenerative medicine of the present invention (gelation step) will be described. The polymer gel in the present invention can be produced by the steps shown in the following first aspect and second aspect. In any of the aspects, the polymer gel can be produced by allowing the solvophilic raw polymers to be crosslinked under conditions less than the overlapping concentration. A polymer gel having a porous structure on the order of μm can be obtained from a solvophilic polymer by conducting the gelation step under the above conditions, which has been previously difficult.

2-a. First Aspect of Process for Producing Polymer Gel

The process for producing a polymer gel in the present invention is characterized by including the following steps in the first aspect:

a) crosslinking solvophilic raw polymers under conditions of less than an overlapping concentration and less than a critical gelation concentration to form gel precursors; and

b) crosslinking the gel precursors with each other using a crosslinking agent to obtain a polymer gel, which is the final target product.

Step a) is a step in which the raw polymers (polymer units) that finally form the polymer gel are reacted in a

state on the verge of gelation to have a structure that does not yet form a gel, in other words, to from gel precursors (polymer clusters) in a sol state. Then, in step b), if desired, these gel precursors are further reacted with each other by adding an appropriate crosslinking agent, and the gel precursors are three-dimensionally crosslinked with each other to obtain a polymer gel, which is the final product. Here, the gel precursors are not necessarily limited to a single type of the identical composition, as described below, but multiple gel precursors having different compositions can also be used. As described above, the production process of the first aspect is based on the concept that the gel precursors are used an intermediate of the "final gel".

In step a), conditions are used in which the starting concentration of the raw polymers is less than the overlapping concentration and less than the critical gelation concentration. The starting concentration of such raw polymers is used, so that it is possible to from a gel precursor of an ungelled sol state that does not lead to gelation, preferably having a structure on the verge of gelation.

In step a), the starting concentration of the raw polymers is less than the overlapping concentration C*, preferably less than 1/3 of the overlapping concentration C*. Here, the "overlapping concentration" (also referred to as "mutually overlapping concentration") is the concentration at which the polymers in the solvent start to spatially come into contact with each other. Generally, the overlapping concentration C* is expressed by the following Expression:

[Expression 1]

$$C^* = 3M_w / (4\pi \cdot \alpha \cdot N_A \cdot R_g^3)$$

wherein $M_w$ is a weight average molecular weight of the polymer, $\alpha$ is a specific gravity of the solvent, $N_A$ is an Avogadro constant, and $R_g$ is a radius of inertia of the polymer.

For the calculation method of the overlapping concentration C*, reference can be made, for example, to Polymer Physics (written by M. Rubinstein and R. Colby). Specifically, for example, it can be determined by viscosity measurement of a dilute solution using the Flory-Fox equation.

Further, in step a), the starting concentration of the raw polymers is set at less than the critical gelation concentration. Here, the "critical gelation concentration" means the minimum concentration of raw polymers necessary to achieve gelation in a system that constructs a gel of a three-dimensional structure by crosslinking of raw polymers, and also called the minimum gelation concentration. The term critical gelation concentration in the present invention also includes when the concentration of only one type of raw polymer is low, that is, when gelation is not induced due to a non-equivalent ratio of each raw polymer, in addition to when the concentrations of all raw polymers do not reach the gelation concentration, for example, in a system that uses two or more types of raw polymers.

Generally, the critical gelation concentration (minimum gelation concentration) depends on the types of raw polymers used, but such concentrations are known in this technical field or can be ascertained easily by experimentation by those skilled in the art. Typically, the critical gelation concentration is from 0.5 to 5 wt%, and the lower limit is about 1/5 of the overlapping concentration.

For example, as described above, when two types of polymer units having nucleophilic functional groups or electrophilic functional groups are used, for example, a low-concentration condition that includes equivalent amounts of these units but overall is not sufficient to achieve gelation can be used, or a condition in which the concentration of one type of polymer unit is low, that is, no gel is produced due to non-equivalent amounts, can be used as the method of adjusting the starting concentration of raw polymers to a condition less than the critical gelation concentration.

Step a) typically can be carried out by mixing or applying stimulation to a solution containing two types of raw polymers. The step can also be carried out by radical polymerization of monomers using a radical initiator. The concentration of each solution, addition rate, mixing rate, and mixing ratio are not particularly limited and can be adjusted as appropriate by those skilled in the art. Even when three or more types of raw polymers are used, it will be obvious that solutions containing the corresponding raw polymers can be prepared and mixed as appropriate in a similar manner. Water, alcohols such as ethanol, DMSO, and the like can be used as the solvent of the solution containing the raw polymers. When the solution is an aqueous solution, an appropriate pH buffer solution such as a phosphate buffer solution can be used.

A two-solution mixing syringe such as that disclosed, for example, in WO2007/083522 can be used as a mixing unit. The temperature of the two solutions during mixing is not particularly limited and should be a temperature that dissolves each of the precursor units and creates a state in which each of the solutions is fluid. An example of the solution temperature during mixing is a range of 1°C to 100°C. The temperature of the two solutions may differ, but it is preferable for the ease of mixing the two solutions that the temperature be the same.

The gel precursors obtained in step a) are formed under conditions that do not yet attain gelation even though the gel precursors have a structure in which precursor units are bonded or crosslinked with each other. The gel precursors have a relationship of G' < G" between the storage modulus G' and the loss modulus G". The value of the loss modulus

G" is known to generally be higher than the storage modulus G' in a polymer before gelation, and magnitude of these physical property values reverses, with G' becoming higher, as gelation occurs thereafter. The point where G' = G "is the so-called gelation point. Therefore, the fact that G' < G" in the gel precursor clusters means that the gel precursor clusters are in a sol state, and a state that has not yet gelled. Preferably, G' < G" < 100 G' at a frequency of 1 Hz.

Preferably, G" of the gel precursors is in a range of 0.005 to 5 Pa, more preferably 0.01 to 1 Pa, and still more preferably 0.01 to 0.5 Pa at a frequency of 1 Hz. These moduli of elasticity can be calculated by a known method such as dynamic viscoelasticity measurement using a known measurement device such as a rheometer.

The gel precursor in the present invention preferably has a diameter of 10 to 1000 nm, more preferably 50 to 200 nm. Preferably, it is desired that the abundance ratio of the gel precursor having a diameter of about 100 nm is the largest in the distribution.

The raw polymers used to form the gel precursor clusters are polymers that are hydrophilic, i.e., soluble in water as a solvent contained in the gel. A detailed description thereof is as described for the polymer units in the polymer gel of the present invention described above.

Next, in step b), the gel precursors obtained in step a) are further reacted with each other and three-dimensionally crosslinked with each other to obtain a polymer gel as the final product. As described above, since the gel precursors are formed so as to be in a state before the gelation point, the substituents used for crosslinking remain in an unreacted state in each of the gel precursors. Therefore, the final gel is formed by crosslinking by reacting these substituents in the gel precursors with residual substituents in other gel precursors.

The polymer gel finally obtained in step b) includes a structure having two regions: a first region in which the polymer units derived from the raw polymers are densely present and a second region in which the polymer units derived from the raw polymers are sparsely present. Accordingly, the fact that a polymer gel having a porous structure on the order of μm can be obtained from solvophilic raw polymers is as described in the description of the polymer gel according to the present invention.

Preferably, in step b), a crosslinking agent for crosslinking the gel precursors with each other can be added or stimulation can be applied. One having substituents the same as the crosslinking groups in the raw polymers can be used as such a crosslinking agent. The raw polymers themselves can also be used as a crosslinking agent, and can be additionally added. For example, when gel precursors are obtained by reacting non-equivalent amounts of two types of raw polymers having nucleophilic functional groups or electrophilic functional groups in step a), the gel precursors can be crosslinked with each other by adding a crosslinking agent having functional groups of the lower concentration. Bis(sulfosuccinimidyl)glutarate ($BS_2G$), DL-dithiothreitol (DTT), a synthetic peptide having a thiol group at an end, or the like can be used as such a crosslinking agent. Further, functional groups (maleimide groups, etc.) can be irradiated with ultraviolet light, for example, to cause photodimerization as a stimulation for crosslinking.

In a preferred aspect, step b) can also be conducted in the presence of a non-reactive polymer having no functional group capable of crosslinking with the gel precursor in the molecule. Such a non-reactive polymer is suitable for obtaining a three-dimensional structure such as a two-phase separation, like a dense phase and a dilute phase. Examples of non-reactive polymers include polymers that have identical basic backbones as the raw polymers, but do not have any crosslinking reactive group in a side chain or at an end, such as polyethylene glycol having no crosslinking reactive group. Alternatively, a material such as cellulose or modified cellulose can be used.

Other reaction solution conditions and the like in step b) are similar to those in step a). Preferably, in step b), the final gel can be obtained in a reaction time of 2 hours or less, preferably 1 hour or less. In general, when producing a gel containing a low concentration of a polymer, a long reaction time is required (depending on the system, for example, about 8 hours when the polymer content is 1 wt% or less). Meanwhile, in the above-described production process, the gel can be produced in a much shorter time.

2-b. Second Aspect of Process for Producing Polymer Gel

The process for producing a polymer gel in the present invention is characterized by including the following steps in the second aspect:

c) crosslinking solvophilic raw polymers having a concentration of less than an overlapping concentration and equal to or more than a critical gelation concentration with each other in the presence of a non-reactive polymer having a predetermined concentration to obtain the polymer gel.

Specifically, in the production process of the second aspect, a non-reactive polymer having a predetermined concentration is added to a solution, so that the polymer gel in the present invention can be obtained in one step, without passing through a gel precursor stage from raw polymer in the first aspect. Even in this case, a raw polymer having a concentration less than the overlapping concentration is used, so that it is possible to obtain a polymer gel having two regions: a dense phase and a dilute phase and having a porous structure on the order of μm.

Here, the non-reactive polymer is a polymer that does not have any functional group capable of crosslinking with the raw polymer in the molecule. Examples of non-reactive polymers include polymers that have identical basic

backbones as the raw polymers, but do not have any crosslinking reactive group in a side chain or at an end, such as polyethylene glycol having no crosslinking reactive group. Alternatively, a material such as cellulose or modified cellulose can be used. Preferably, the concentration of the non-reactive polymer is from 1/10 to 10, and more preferably from 1/5 to 2 of the concentration of the raw polymers.

Further, the starting concentration of the raw polymers in the second aspect is the same as that in the first aspect in that the starting concentration is less than the overlapping concentration. Meanwhile, the second aspect is different from the first aspect in that the starting concentration of the raw polymer is equal to or more than the critical gelation concentration and gelation is conducted in one step.

In addition, the reaction solution conditions, the mixing method, and the like are similar to those in the first aspect.

2. Kit of Present Invention

In another aspect, the present invention relates to a kit for producing a gel material for regenerative medicine. The kit is based on the above-described process for producing a polymer gel.

Specifically, in accordance with the aspect of the process for producing a polymer gel described above, the following kits of the first aspect and the second aspect can be mentioned (although the order is reversed, preferred aspects of these kits correspond to the second aspect and the first aspect relating to the production process described above, respectively). However, it should be understood that the polymer gel used in the gel material for regenerative medicine can be produced without using these kits.

In the first aspect, the kit of the present invention includes the following two types of solutions (A) and (B) stored without being mixed with each other:

(A) an aqueous solution containing a hydrophilic first raw polymer having a concentration of less than an overlapping concentration and equal to or more than a critical gelation concentration; and

(B) an aqueous solution containing a hydrophilic second raw polymer having a concentration of less than an overlapping concentration and equal to or more than a critical gelation concentration.

In addition, in the first aspect, the kit of the present invention further includes, in the solution (A) and/or the solution (B), a non-reactive polymer having no functional group capable of crosslinking with the raw polymer in the molecule.

As described above, the polymer gel obtained using the kit of the present invention has the structural characteristics below. That is, a polymer gel obtained by mixing the solutions (A) and (B) is a polymer gel in which the first raw polymer and the second raw polymer are crosslinked with each other, wherein the polymer gel has a structure including two regions: a first region in which polymer units derived from the raw polymers are densely present and a second region in which polymer units derived from the raw polymers are sparsely present.

In the second aspect of the kit of the present invention (corresponding to the first aspect of the production process), the kit includes the following two types of solutions (A') and (B') stored without being mixed with each other:

(A') an aqueous solution containing a first gel precursor, wherein the first gel precursor is obtained by crosslinking hydrophilic raw polymers under conditions of less than an overlapping concentration and less than a critical gelation concentration, and the first gel precursor has a relationship of $G' < G''$ between a storage modulus $G'$ and a loss modulus $G''$; and

(B') an aqueous solution containing a second gel precursor, wherein the second gel precursor is obtained by crosslinking hydrophilic raw polymers under conditions of less than an overlapping concentration and less than a critical gelation concentration, and the second gel precursor has a relationship of $G' < G''$ between a storage modulus $G'$ and a loss modulus $G''$.

In addition, in the second aspect, the kit of the present invention further includes, in the solution (A') and/or the solution (B'), a non-reactive polymer having no functional group capable of crosslinking with the raw polymer in the molecule.

Similarly to the first aspect, the polymer gel obtained using the kit of the present invention has the structural characteristics below. That is, a polymer gel obtained by mixing the solutions (A') and (B') is a polymer gel in which the first raw polymer and the second raw polymer are crosslinked with each other, wherein the polymer gel has a structure including two regions: a first region in which polymer units derived from the raw polymers are densely present and a second region in which polymer units derived from the raw polymers are sparsely present.

Preferred aspects of the raw polymers used in the kit of the present invention are the same as those of the above-mentioned polymer units. For example, preferably, the raw polymers in the kit of the present invention are polymers having a polyethylene glycol backbone or a polyvinyl backbone. Similarly, in the kit of the present invention, the raw

polymers include a first polymer having one or more nucleophilic functional groups in a side chain or at an end and a second polymer having one or more electrophilic functional groups in a side chain or at an end. Here, the types of the nucleophilic and electrophilic functional groups are also as described above.

In the second aspect of the kit of the present invention, preferably, the first gel precursor and the second gel precursor both have a first polymer having one or more nucleophilic functional groups in a side chain or at an end and a second polymer having one or more electrophilic functional groups in a side chain or at an end, the first gel precursor has a higher first polymer content than a second polymer content, and the second gel precursor has a higher second polymer content than a first polymer content. Furthermore, the gel precursor preferably has a diameter of 10 to 1000 nm, more preferably 50 to 200 nm.

The non-reactive polymer included in the kit of the present invention can be, for example, polyethylene glycol or cellulose, which has no crosslinking reactive group.

The solutions (A), (B), (A') and (B') in the kit of the present invention can contain, as another component, an appropriate pH buffer solution such as a phosphate buffer solution.

3. Treatment Process of Present Invention

As described above, the gel material for regenerative medicine of the present invention can be used in regenerative medicine applications, and is preferably suitable for tissue regeneration. Therefore, the present invention also relates to a treatment process (or tissue regeneration process) using the gel material for regenerative medicine.

The "tissue" to be a target of the treatment process of the present invention (or tissue regeneration process) is not particularly limited as long as it is a biological tissue requiring regeneration or repair, and examples thereof include bone, cartilage, skin, nerve, organ, and the like. Therefore, the treatment process of the present invention is preferably directed to the regeneration of bone, cartilage, skin or nerve, more preferably to the regeneration of bone.

In the treatment process of the present invention (or tissue regeneration process), specifically, it is preferable to cover an affected part requiring regeneration or repair with a gel material for regenerative medicine by administering, injecting, or applying the gel material for regenerative medicine to the affected part (hereinafter, referred to as "administration and the like"). Alternatively, the space (site to be regenerated or repaired) in the affected part can be filled with the gel material for regenerative medicine. For example, in the treatment of torn or damaged bone or nerve, it is assumed that the gel material for regenerative medicine is administered to an affected part which has been subjected to treatment such as rejoining of the torn or damaged portion. In some cases, creating of an environment in which the gel material for regenerative medicine is present in the peripheral region of the affected part by such administration and the like is also included.

In the treatment process of the present invention (or tissue regeneration process), when the gel material for regenerative medicine is administered and the like, the gel material for regenerative medicine can be produced in situ (in vivo) in or around the affected part in need thereof. In this case, it is preferable to mix two types of polymer solutions (e.g., solutions (A) and (B) or solutions (A') and (B')) in or around the affected part based on the above-described process for producing a polymer gel using the kit of the present invention.

Therefore, the present invention provides a process for producing a gel material for regenerative medicine in vivo using the kit. From another point of view, the present invention also provides use of the kit in tissue regeneration treatment.

When the gel material for regenerative medicine is produced in situ (in vivo) in or around an affected part, the gelation time can be preferably within 1 hour, more preferably within 10 minutes, and still more preferably within 1 minute. As described above, the gelation time can be adjusted by appropriately setting the polymer concentration, pH, and ionic strength of the polymer solutions included in the kit.

Examples

Hereinafter, the present invention will be described in more detail with reference to Examples, but the present invention is not limited thereto. In the following experiments, the $^1$H NMR spectrum was analyzed using JNM-ECS400 (400 MHz), manufactured by JEOL Ltd. Deuterated chloroform was used as a solvent, and tetramethylsilane was used as an internal standard. The molecular weight was determined using the linear positive ion mode of an Ultraflex III mass spectrometer made by Brucker Daltonics, Inc. In the following examples, the unit of polymer concentration (g/L) is used, which corresponds to 1 g/L = about 0.1 wt%.

[Example 1]

Synthesis of Gel Precursors [SHPEG + MAPEG]

A gel precursor serving as a precursor in a gelation reaction was synthesized in the following manner using tetrathiol-polyethylene glycol (SHPEG) having a -SH group at the end and tetramaleimidyl-polyethylene glycol (MAPEG) having a maleimidyl group at the end. As these raw polymers SHPEG and MAPEG, those commercially available from NOF CORPORATION. were used (both had a molecular weight of 10,000).

Two 50 mM citric acid-phosphate buffer solutions, containing SHPEG and MAPEG, respectively, were separately prepared (amount ratio of the materials: SHPEG/MAPEG = 1/1, overall polymer concentration: 20 g/L). The two solutions thus obtained were mixed in another container, and defoamed and stirred with a planetary centrifugal mixer. Then, the mixed solution was quickly transferred to a Falcon tube. The tube was capped to prevent drying and allowed to stand at room temperature for 12 hours. At this time, a plurality of samples containing two types of gel precursors was prepared so that an excess of either of SHPEG and MAPEG was a molar ratio of SHPEG : MAPEG of 0.78 : 0.22 or 0.22 : 0.78.

A rheometer was used to observe the temporal changes in storage modulus G' and loss modulus G" in the reaction step (at 25°C and 1 Hz). It was confirmed that the obtained gel precursor had a relationship of G' < G" at the end point of the reaction, and these were polymer clusters in a sol state that had not yet formed a gel.

[Example 2]

Synthesis of Polymer Gel

A polymer gel (Oligo-TetraPEG gel) was synthesized in the following manner using the gel precursor synthesized in Example 1.

The gel precursor solution A (r = 0.78) and the gel precursor solution B (r = 0.22) obtained in Example 1 were respectively diluted with water to 10 g/L (or 20 g/L). The amount of unreacted SH groups in each of the solutions was calculated, a crosslinking agent (bis-(sulfosuccinimidyl)glutarate ($BS_2G$)) was added in an equal amount to the above amount, and the resultant mixture was defoamed and stirred with a planetary centrifugal mixer. Then, the mixed solution was quickly transferred to a Falcon tube. The tube was capped to prevent drying and allowed to stand at room temperature for 5 hours. Observation was conducted with a rheometer (at 25°C and 1 Hz), and it was confirmed that the solution had a relationship of G' > G" at the end point of the reaction, and a polymer gel was formed by crosslinking of the gel precursors (Fig. 1).

[Example 3]

Transmittance of Polymer Gel

Fig. 2 shows changes in transmittance in the gelation step of Example 2. The two solutions of the gel precursors were mixed and injected into cuvettes, and the transmittance was calculated from the temporal changes of the absorbance at 25°C and a wavelength of 400 nm. In the figure, changes in G' and G" are also shown, and the point at which G' = G" is the gelation point.

Further, Fig. 3 shows the changes in transmittance and G' and G" for a single-phase polymer gel (Comparative Example) obtained by mixing two solutions: 60 g/L of SHPEG and MAPEG solutions without passing through a gel precursor step.

The comparison between Figs. 2 and 3 showed that, in the normal gel without passing through a gel precursor step (Fig. 3), almost no change in transmittance was observed before and after gelation (T = around 98%), whereas, in the polymer gel of Example 2 (Fig. 2), the transmittance T decreased significantly from about 99% to about 94% with gelation, and the gel became cloudy. This is because, in the polymer gel of the present invention, two regions: a dense phase with the polymer units densely present and a dilute phase with the polymer units sparsely present were generated due to gelation, and phase separation occurred.

[Example 4]

Swelling Test of Polymer Gel

A swelling test was conducted on three types of polymer gels (a and b) of the present invention and a comparative example gel (c). Gels a and b were synthesized according to Example 2.

(a) Oligo-Tetra-PEG gel (10 g/L):
Two types of gel precursors were produced at 20 g/L, r = 0.78; 20 g/L, r = 0.22 according to Example 2. Each of the gel precursor solutions was diluted to 10 g/L and the two solutions were mixed to form a gel.
(b) Oligo-Tetra-PEG gel (20 g/L):
Two types of gel precursors were produced at 20 g/L, r = 0.78, 20 g/L, r = 0.22 according to Example 2. Each of the gel precursor solutions is directly mixed to form a gel.
(c) Tetra-PEG gel (Comparative Example):
60 g/L of SHPEG and MAPEG solutions were mixed to form a gel.

A cylindrical sample (height 7 mm, diameter 15 mm) was produced. After the gelation reaction was sufficiently completed (1 day), the sample was immersed in pure water and the temporal changes of degree of swelling were measured at 25°C. The results obtained are shown in Fig. 4.

In the normal gel c without passing through a gel precursor step, swelling of the gel was observed over time. Meanwhile, the polymer gel of the present invention showed characteristic properties of shrinking with long relaxation times.

[Example 5]

Osmotic Pressure Test of Polymer Gel

The osmotic pressure ($\Pi_{os}$) and elastic pressure ($\Pi_{el}$) of the polymer gel of the present invention produced according to Example 2 were measured. As Comparative Examples, the raw polymer before gelation and the normal gel without passing through a gel precursor step were measured in a similar manner to the above measurement.

- Polymer gel of the present invention:
  According to Example 2, gels produced under a plurality of conditions with gel precursor solution concentrations of 10, 12.5, 15, 17.5, and 20 g/L were used.

- Raw polymer:
  10, 20, 30, 40, 50, and 60 g/L of MAPEG solutions were used.
- Comparative example gel:
  10, 20, 30, 40, 50, or 60 g/L of SHPEG and MAPEG solutions were mixed and gelled.

A sample was produced in a dialysis membrane and dialyzed against a PVP solution (29 k, 5 to 120 g/L), and the concentration of PVP at which the sample did not swell from the initial state was determined. At that point, the swelling pressure of the gel ($\Pi_{os}$ - $\Pi_{el}$) and the osmotic pressure of PVP ($\Pi_{PVP}$) are balanced. Separately, the elastic pressure ($\Pi_{el}$ = G') was measured with a rheometer, and $\Pi_{os}$ was calculated using the following Expression:

[Expression 2]

$$\Pi_{os} = \Pi_{PVP} + \Pi_{el}$$

Fig. 5 shows a plot of the osmotic pressure ($\Pi_{os}$) obtained at each gelation concentration (C). As a result, it was found that the polymer gel of the present invention had an osmotic pressure lower than both the raw polymer and the comparative example gel having identical concentrations in the overlapping concentration (C*) or less.

[Example 6]

Fluorescence Microscope Images of Polymer Gel

The structures of the gel of the present invention (a) and the comparative example gel (b) were observed using a two-photon laser microscope (Zeiss).

(a) Oligo-Tetra-PEG gel (10 g/L):
Two types of gel precursors were produced at 20 g/L, r = 0.78; 20 g/L, r = 0.22 according to Example 2. Each of the gel precursor solutions was diluted to 10 g/L and the two solutions were mixed to form a gel.
(b) Tetra-PEG gel (Comparative Example):

60 g/L of SHPEG and MAPEG solutions were mixed to form a gel.

After preparing the gel samples (a) and (b), the gel samples were allowed to stand in water for 7 days.

Primary staining: the gel samples were immersed in an anti-PEG solution (0.04 g/L), allowed to stand for 1 hour, and washed for 15 minutes three times.

Secondary staining: the gel samples were immersed in antibody-AlexaFloar488 (0.04 g/L), allowed to stand for 1 hour, and washed for 15 minutes three times.

Fig. 6 shows the obtained fluorescence microscope images. It was found that the Tetra-PEG gel (left figure) of the comparative example had a network structure on the order of nm, whereas the polymer gel of the present invention (right figure) had a structure on the order of μm much larger than the structure predicted from the molecular structure (porous structure). The network structure on the order of μm has an outer circumference formed by a dense phase with polymers densely present, and the inside thereof is a dilute phase or a solvent.

[Example 7]

Adhesion of Cells to Polymer gel

The adhesion of cells to the polymer gel of the present invention produced in Example 2 was also evaluated. As a result, it was confirmed that the polymer gel of the present invention was able to adhere ATDC5 (somatic pluripotent cells) more efficiently than the normal Tetra-PEG gel (Comparative Example) without passing through a gel precursor step.

[Example 8]

Application to Bone Regeneration

(1) Test in Rat Tibial Bone Defect Model

Using a rat tibial bone defect model, the effect of the polymer gel having a sponge-like porous structure of the present invention on bone regeneration was tested. A 9 mm bone defect was formed in the adult rat tibia, and the bone defect site was filled with a polymer gel (Oligo-TetraPEG gel) by the process of Example 2. Specifically, polymer solutions containing SHPEG and MAPEG, respectively, were injected into the bone defect site and the solutions were mixed therein, followed by gelation in situ.

Thereafter, the tissue around the defect was sutured, and the bone regeneration effect at the bone defect was observed over time (Fig. 7). As a result of the micro-CT analysis, 4 weeks after the bone defect formation , in the polymer gel-unfilled group (control), bone regeneration was incomplete, and bone union between the ends of the bone defect was not observed (a to c of Fig. 7A). Meanwhile, in the polymer gel-filled group, bone union between the ends of the bone defect was observed 2 weeks after the bone defect formation, and most of the bone defect sites were replaced with new bone after 4 weeks (d to f in FIG. 7A). As a result of histological analysis of the bone defect site 4 weeks after the bone defect formation, in the control group, infiltration of fibroblasts was observed in the bone defect site. Non-union of the defected bone ends was observed, and bone regeneration was not sufficient (g and h in Fig. 7B). Meanwhile, in the polymer gel-filled group, most of the defect sites were replaced with new bone, and union of the bone ends was observed (i and j in Fig. 7B).

(2) Test in Mouse Cranial Bone Defect Model

Next, a 4-mm bone defect was formed in the adult mouse cranium, and then the effect of the polymer gel (Oligo-TetraPEG gel) was tested in a similar manner to (1) above. In this analysis, 1 day after the bone defect was produced and the tissue around the defect was sutured, the polymer gel obtained by mixing two polymer solutions containing SHPEG and MAPEG, respectively, was filled in the defect site. For comparison, the comparative example gel ("c) Tetra-PEG gel" of Example 4) was used, in addition to the sham group (negative control; NTC). As a result of micro-CT analysis shown in Fig. 8, in the sham group and the comparative example gel group, bone regeneration was hardly observed 12 weeks after the bone defect, whereas in the Oligo-TetraPEG gel group of the present invention ("Tetra-PEG sponge" group in Fig. 8), a remarkable bone regeneration effect was observed.

[Example 9]

Subcutaneous Application

A subcutaneous implantation test in rats was conducted on two types of polymer gels: a gel of the present invention (a) and a comparative example gel (b).

(a) Oligo-Tetra PEG gel:
Two types of gel precursors were produced at 20 g/L, r = 0.78; 20 g/L, r = 0.22 according to Example 2. Each of the gel precursor solutions was diluted to 10 g/L and the two solutions were mixed to form a gel.
(b) Tetra PEG gel (Comparative Example):
20 g/L of SHPEG and MAPEG solutions were mixed to form a gel.

The back of the rat was shaved under anesthesia, and a total of two incision lines were made on the right and left of the back to create pockets subcutaneously. The gel of the present invention (a) was filled in the right side, and the comparative example gel (b) was filled in the left side. Specifically, polymer solutions containing SHPEG and MAPEG, respectively, were injected into the right-side or left-side and the solutions were mixed therein, followed by gelation in situ.
The skin on the wound surface was then sutured. 2 weeks after treatment, rats were sacrificed and implants were removed, and evaluation was performed macroscopically (Fig. 9). In the gel of the present invention (a), it was found that soft tissues were formed in the gel. Meanwhile, in the comparative example gel (b), no neoplasia was observed.
The above results demonstrate that the regeneration and repair of biological tissues can be promoted by applying the gel material for regenerative medicine containing the polymer gel having a sponge-like porous structure of the present invention to an affected part.

**Claims**

1. A gel material for regenerative medicine comprising a polymer gel in which hydrophilic polymer units are crosslinked with each other,
wherein the polymer gel contains water as a solvent and has a three-dimensional network structure having two regions: a first region in which the polymer units are densely present and a second region in which the polymer units are sparsely present, and a mesh size composed of the first region is the range of 1 to 500 $\mu$m.

2. The gel material for regenerative medicine according to claim 1, wherein the gel material has a lower transmittance than a transmittance of the polymer units before gelation.

3. The gel material for regenerative medicine according to claim 1 or 2, wherein the gel material has an osmotic pressure of 1/5 to 1/2 of an osmotic pressure of the polymer units before gelation.

4. The gel material for regenerative medicine according to any one of claims 1 to 3, wherein an osmotic pressure ($\Pi_{os}$) and an elastic pressure ($\Pi_{el}$) after a lapse of a certain period of time from gelation have a relationship of $\Pi_{el} > \Pi_{os}$.

5. The gel material for regenerative medicine according to any one of claims 1 to 4, wherein a polymer concentration in the first region is from 10 to 99 wt%, and a polymer concentration in the second region is from 0 to 1 wt%.

6. The gel material for regenerative medicine according to any one of claims 1 to 5, wherein the polymer gel has a polymer content of 5 wt% or less.

7. The gel material for regenerative medicine according to any one of claims 1 to 6, wherein the polymer units are polymers having a polyethylene glycol backbone or a polyvinyl backbone.

8. The gel material for regenerative medicine according to any one of claims 1 to 7, wherein the polymer units comprise a first polymer unit having one or more nucleophilic functional groups in a side chain or at an end and a second polymer unit having one or more electrophilic functional groups in a side chain or at an end.

9. The gel material for regenerative medicine according to claim 8, wherein the nucleophilic functional groups are selected from the group consisting of a thiol group and an amino group, and the electrophilic functional groups are selected from the group consisting of a maleimidyl group, an N-hydroxy-succinimidyl (NHS) group, a sulfosuccinimidyl

group, a phthalimidyl group, an imidazolyl group, an acryloyl group, a nitrophenyl group, and $-CO_2PhNO_2$.

**10.** The gel material for regenerative medicine according to any one of claims 1 to 9, which is used for regeneration of a tissue.

**11.** The gel material for regenerative medicine according to claim 10, wherein the tissue is bone, cartilage, skin, or nerve.

**12.** A treatment process using the gel material for regenerative medicine according to any one of claims 1 to 11.

**13.** A kit for producing a gel material for regenerative medicine, the kit comprising the following two types of solutions (A) and (B) stored without being mixed with each other:

(A) an aqueous solution containing a hydrophilic first raw polymer having a concentration of less than an overlapping concentration and equal to or more than a critical gelation concentration; and
(B) an aqueous solution containing a hydrophilic second raw polymer having a concentration of less than an overlapping concentration and equal to or more than a critical gelation concentration;

wherein the solution (A) and/or the solution (B)further contains a non-reactive polymer having no functional group capable of crosslinking with the raw polymer in the molecule,

a polymer gel obtained by mixing the solutions (A) and (B) is a polymer gel in which the first raw polymer and the second raw polymer are crosslinked with each other, and
the polymer gel has a structure including two regions: a first region in which polymer units derived from the raw polymers are densely present and a second region in which polymer units derived from the raw polymers are sparsely present.

**14.** A kit for producing a gel material for regenerative medicine, the kit comprising the following two types of solutions (A') and (B') stored without being mixed with each other:

(A') an aqueous solution containing a first gel precursor, wherein the first gel precursor is obtained by crosslinking hydrophilic raw polymers under conditions of less than an overlapping concentration and less than a critical gelation concentration, and the first gel precursor has a relationship of $G' < G''$ between a storage modulus G' and a loss modulus G"; and
(B') an aqueous solution containing a second gel precursor, wherein the second gel precursor is obtained by crosslinking hydrophilic raw polymers under conditions of less than an overlapping concentration and less than a critical gelation concentration, and the second gel precursor has a relationship of $G' < G''$ between a storage modulus G' and a loss modulus G";
wherein the solution (A') and/or the solution (B')further contains a crosslinking agent,
a polymer gel obtained by mixing the solutions (A') and (B') is a polymer gel in which the first raw polymer and the second raw polymer are crosslinked with each other, and
the polymer gel has a structure including two regions: a first region in which polymer units derived from the raw polymers are densely present and a second region in which polymer units derived from the raw polymers are sparsely present.

**15.** The kit according to claim 13 or 14, wherein the raw polymers are polymers having a polyethylene glycol backbone or a polyvinyl backbone.

**16.** The kit according to any one of claims 13 to 15, wherein the raw polymers comprise a first polymer having one or more nucleophilic functional groups in a side chain or at an end and a second polymer having one or more electrophilic functional groups in a side chain or at an end.

**17.** The kit according to claim 16, wherein the nucleophilic functional groups are selected from the group consisting of a thiol group and an amino group, and the electrophilic functional groups are selected from the group consisting of a maleimidyl group, an N-hydroxy-succinimidyl (NHS) group, a sulfosuccinimidyl group, a phthalimidyl group, an imidazolyl group, an acryloyl group, a nitrophenyl group, and $-CO_2PhNO_2$.

**18.** The kit according to claim 13, wherein the non-reactive polymer is polyethylene glycol or cellulose, which has no crosslinking reactive group.

**19.** The kit according to claim 14, wherein the first gel precursor and the second gel precursor both have a first polymer having one or more nucleophilic functional groups in a side chain or at an end and a second polymer having one or more electrophilic functional groups in a side chain or at an end, the first gel precursor has a higher first polymer content than a second polymer content, and the second gel precursor has a higher second polymer content than a first polymer content.

**20.** The kit according to claim 14, wherein the gel precursors have a diameter of 10 to 1000 nm.

**21.** A process for producing a gel material for regenerative medicine in vivo using the kit according to any one of claims 13 to 20.

**22.** Use of the kit according to any one of claims 13 to 20 in tissue regeneration treatment.

[FIG. 1]

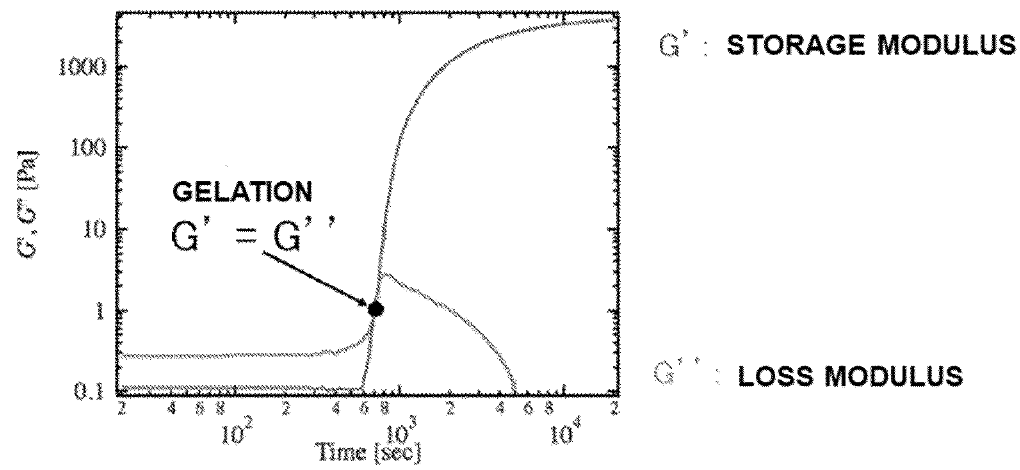
G' : STORAGE MODULUS
G'' : LOSS MODULUS
GELATION
G' = G''
G', G'' [Pa]
Time [sec]

[Fig. 2]

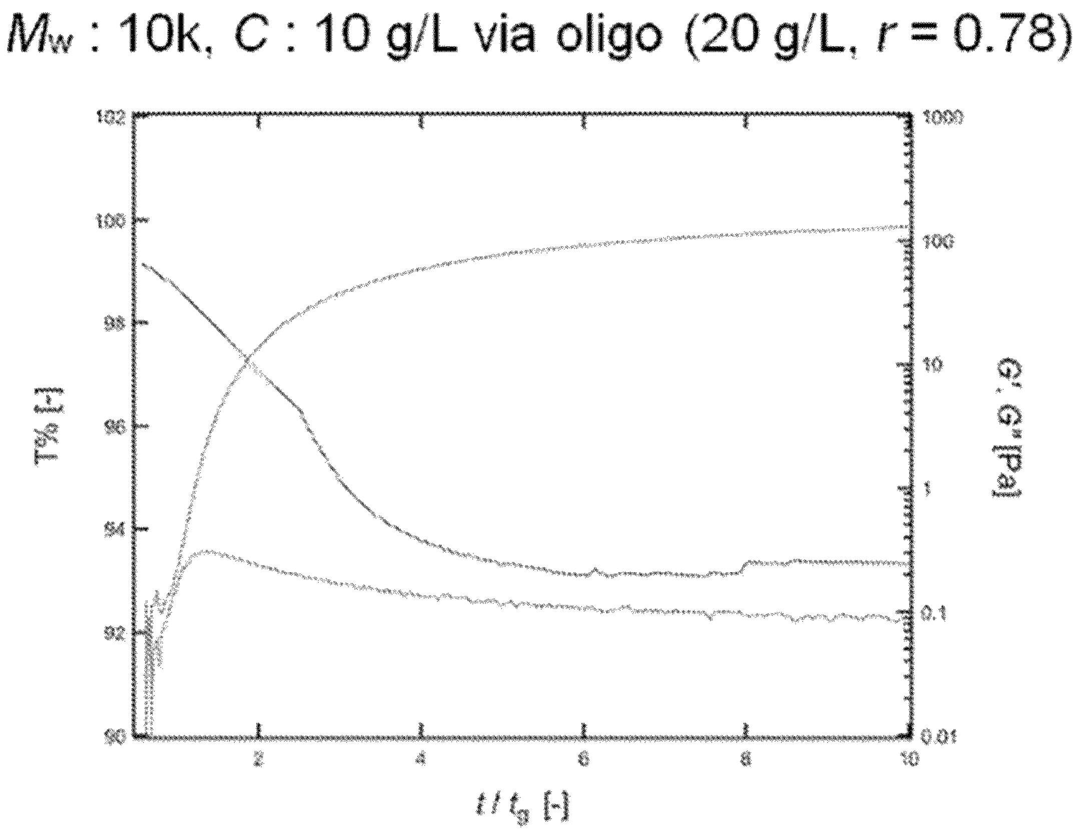
Mw : 10k, C : 10 g/L via oligo (20 g/L, r = 0.78)
T% [-]
G', G" [Pa]
t / tg [-]

[Fig. 3]

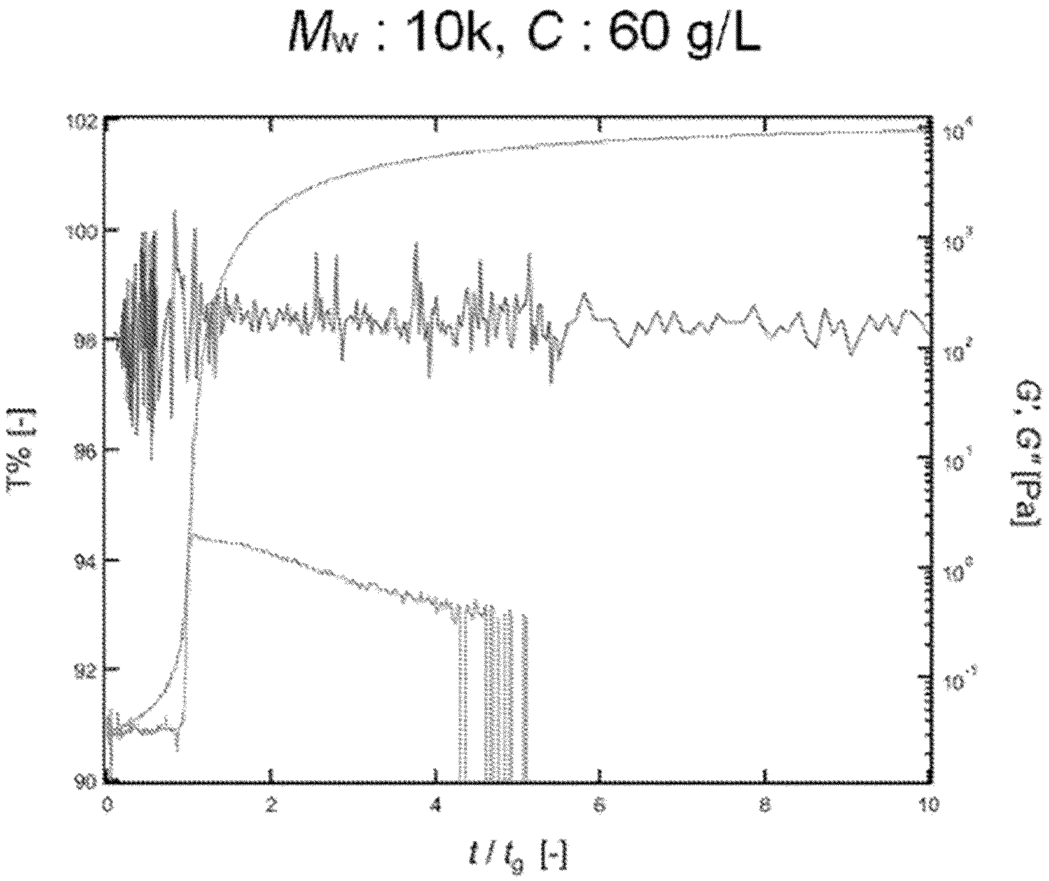

[Fig. 4]

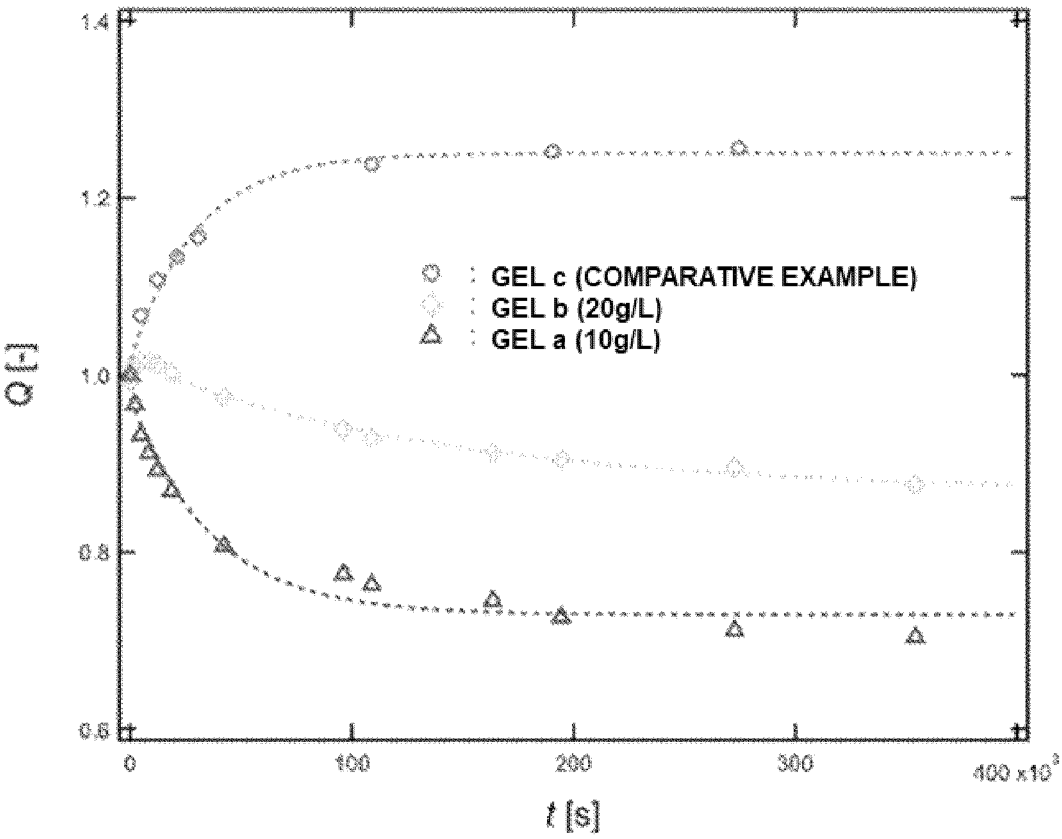
1.4
1.2
1.0
0.8
0.6
Q [-]
0
100
200
300
400 x10³
t [s]
: GEL c (COMPARATIVE EXAMPLE)
: GEL b (20g/L)
: GEL a (10g/L)

[Fig. 5]

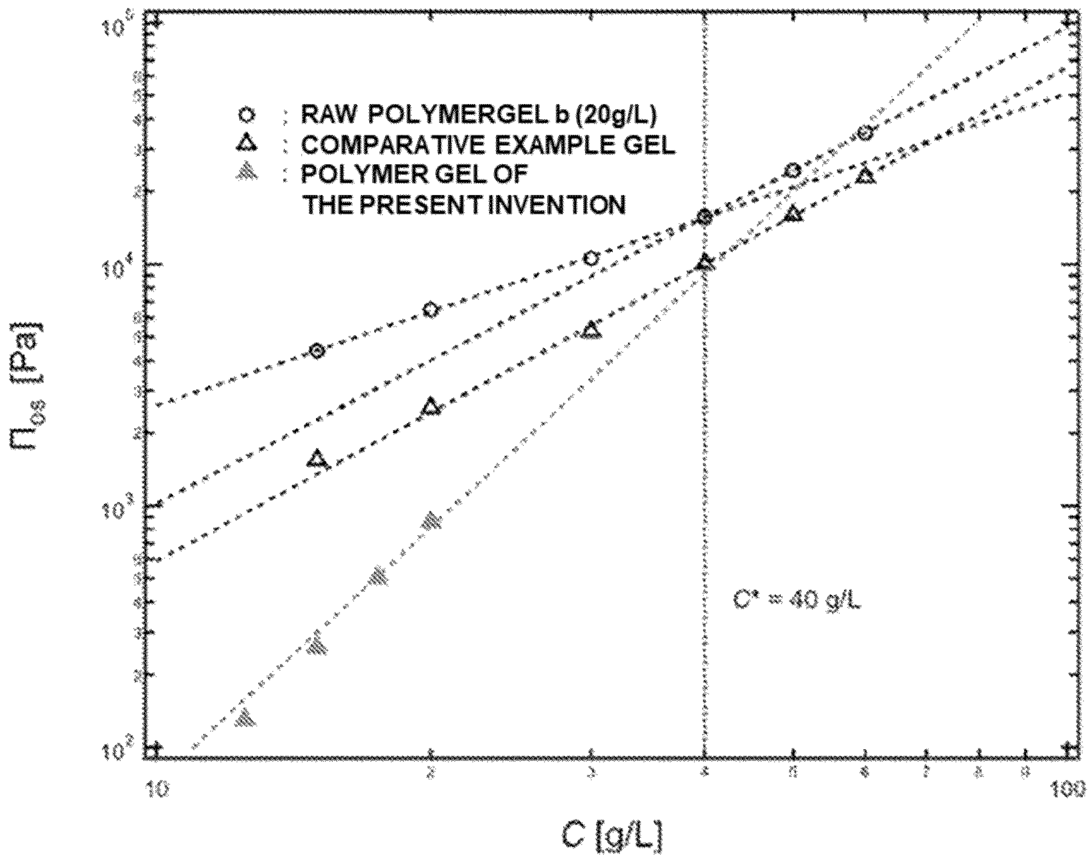
RAW POLYMERGEL b (20g/L)
COMPARATIVE EXAMPLE GEL
POLYMER GEL OF THE PRESENT INVENTION
C* = 40 g/L
Π_os [Pa]
C [g/L]

[Fig. 6]

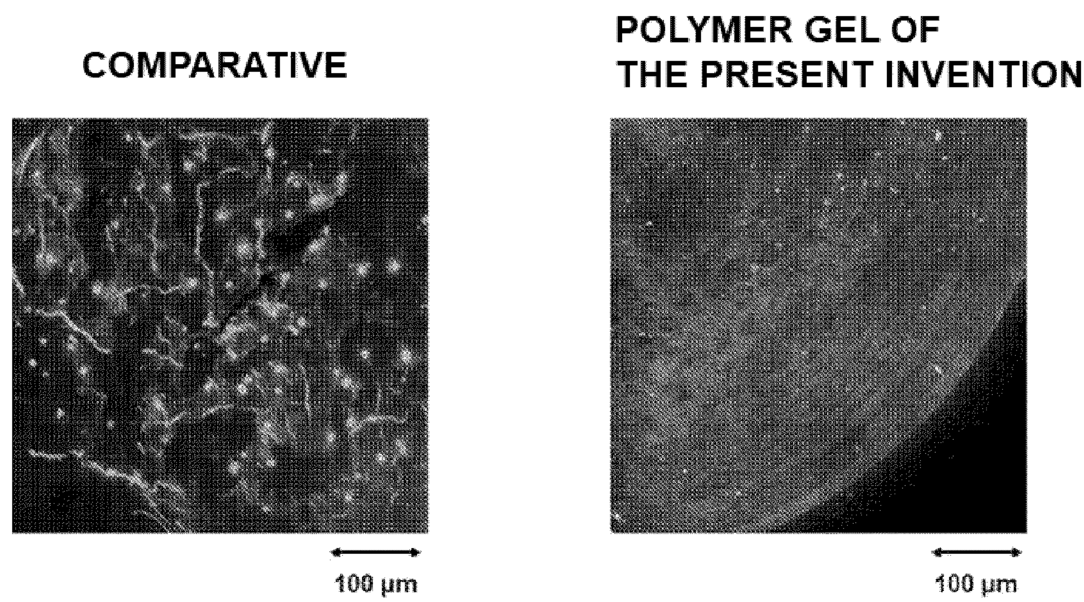
COMPARATIVE
POLYMER GEL OF THE PRESENT INVENTION
100 μm
100 μm

[Fig. 7]

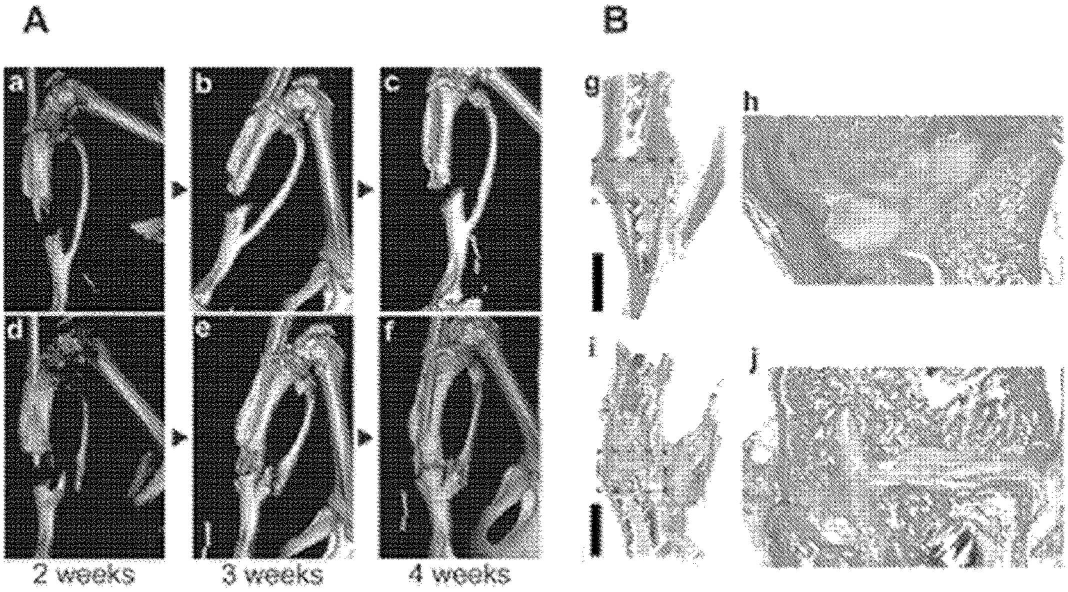
A
B
a
b
c
d
e
f
g
h
i
j
2 weeks
3 weeks
4 weeks

[Fig. 8]

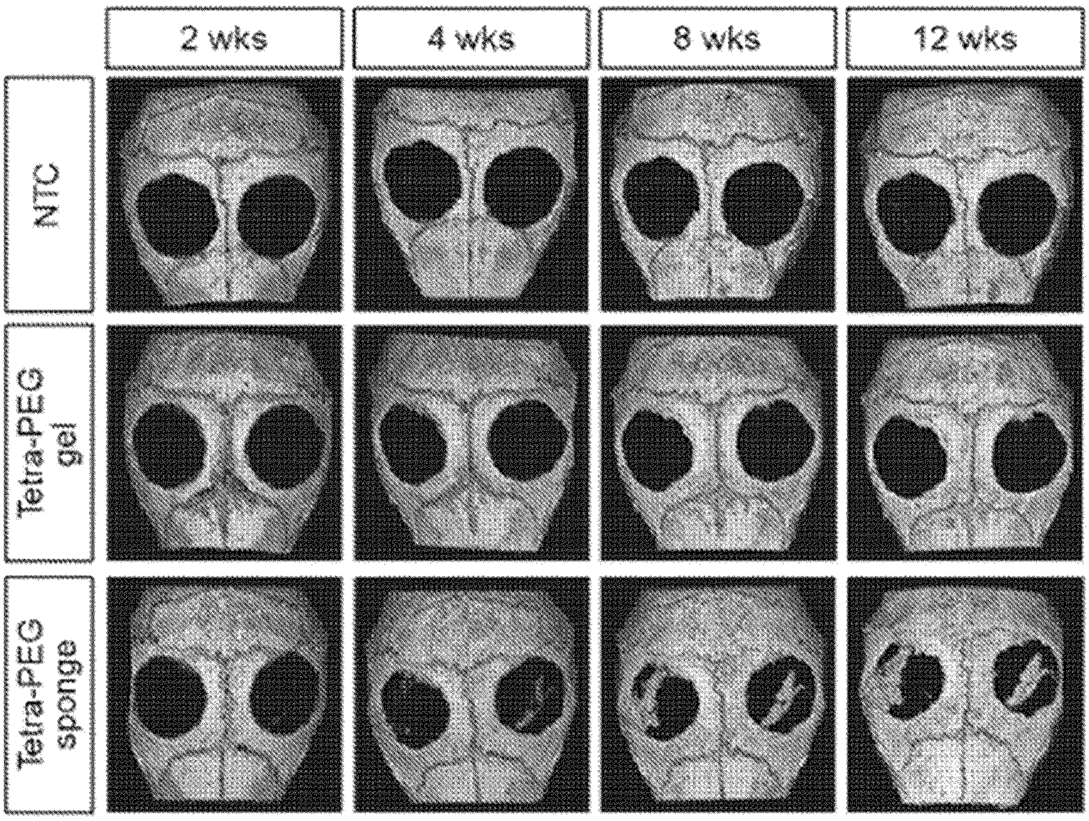
2 wks
4 wks
8 wks
12 wks
NTC
Tetra-PEG gel
Tetra-PEG sponge

[Fig. 9]

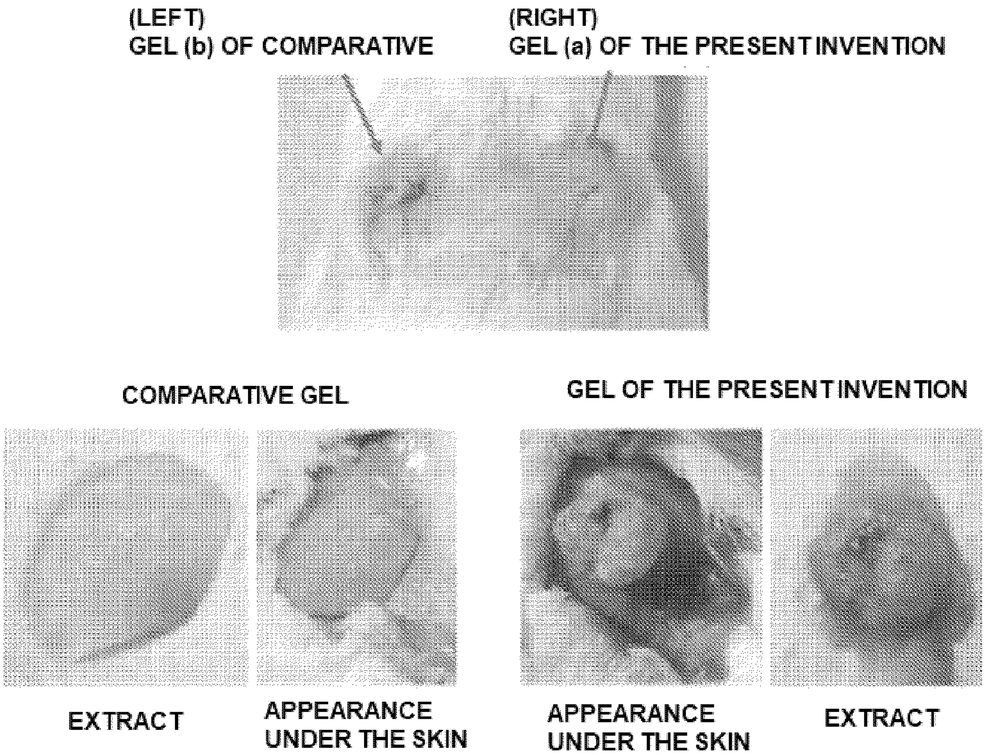
(LEFT)
GEL (b) OF COMPARATIVE
(RIGHT)
GEL (a) OF THE PRESENT INVENTION
COMPARATIVE GEL
GEL OF THE PRESENT INVENTION
EXTRACT
APPEARANCE UNDER THE SKIN
APPEARANCE UNDER THE SKIN
EXTRACT

**INTERNATIONAL SEARCH REPORT**

International application No. PCT/JP2021/002405

A. CLASSIFICATION OF SUBJECT MATTER
A61L 27/18(2006.01)i; A61L 27/52(2006.01)i; A61L 27/54(2006.01)i; A61L 27/56(2006.01)i; A61L 27/60(2006.01)i
FI: A61L27/18; A61L27/54; A61L27/60; A61L27/52; A61L27/56

According to International Patent Classification (IPC) or to both national classification and IPC

B. FIELDS SEARCHED

Minimum documentation searched (classification system followed by classification symbols)
A61L27/18; A61L27/52; A61L27/54; A61L27/56; A61L27/60

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS/WPIDS (STN); PubMed; nature.com

C. DOCUMENTS CONSIDERED TO BE RELEVANT

| Category* | Citation of document, with indication, where appropriate, of the relevant passages | Relevant to claim No. |
|---|---|---|
| Y | WO 2017/119296 A1 (THE UNIVERSITY OF TOKYO) 13 July 2017 (2017-07-13) examples 2, 3, 5, paragraph [0001] | 1-22 |
| Y | 酒井 崇匡、藤藪 岳志，生体内における膨潤挙動が制御可能なインジェクタブルハイドロゲル, Drug Delivery System, 25 July 2019, vol. 34, no. 3, pp. 186-200, https://doi.org/10.2745/dds.34.186, fig. 13, page 199, section "conclusion", (SAKAI, Takamasa, FUJIYABU, Takeshi, "Injectable hydrogel with Controlled Swelling Behavior in vivo") | 1-22 |
| Y | JP 2018-526171 A (The UNIVERSITY OF SYDNEY) 13 September 2018 (2018-09-13) abstract | 1-22 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 March 2021 (04.03.2021) | 16 March 2021 (16.03.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2021/002405

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2013-544248 A (UNIVERSITAETSKLINIKUM FREIBURG) 12 December 2013 (2013-12-12) abstract | 1-22 |
| A | HAYASHI, Kaori et al., "Fast-forming hydrogel with ultralow polymeric content as an artificial vitreous body", Nature Biomedical Engineering, 09 March 2017, vol. 1, Article No. 0044, pp. 1-7, https://cloi.org/10.1038/s41551-017-0044, entire text, all drawings | 1-22 |
| A | 酒井 崇匡，構造明確な高分子ゲルのゲル化過程と力学特性の解明に関する研究，日本レオロジー学会誌，16 December 2019, vol. 47, no. 5, pp. 183-195, https://doi.org/10.1678/rneology_47.183, entire text, all drawings, (SAKAI, Takamasa, "Gelation Process and Mechanical Properties of Polymer Gels with Well-Defined Network Structure", Nihon Reoroji Gakkaishi) | 1-22 |
| A | WO 2014/157186 A1 (THE UNIVERSITY OF TOKYO) 02 October 2014 (2014-10-02) entire text, all drawings | 1-22 |
| A | WO 2016/143647 A1 (THE UNIVERSITY OF TOKYO) 15 September 2016 (2016-09-15) entire text, all drawings | 1-22 |

Form PCT/ISA/210 (continuation of second sheet) (January 2015)

**INTERNATIONAL SEARCH REPORT**
Information on patent family members

International application No.
PCT/JP2021/002405

| Patent Documents referred in the Report | Publication Date | Patent Family | Publication Date |
|---|---|---|---|
| WO 2017/119296 A1 | 13 Jul. 2017 | US 2019/0015559 A1 examples 2, 3, 5, paragraph [0001]<br>EP 3400972 A1 | |
| JP 2018-526171 A | 13 Sep. 2018 | US 2018/0243469 A1 abstract<br>EP 3344304 A1 | |
| JP 2013-544248 A | 12 Dec. 2013 | US 2013/0315996 A1 abstract<br>EP 2455104 A1<br>CN 103249433 A | |
| WO 2014/157186 A1 | 02 Oct. 2014 | (Family: none) | |
| WO 2016/143647 A1 | 15 Sep. 2016 | US 2018/0030205 A1<br>EP 3269755 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007083522 A


### Non-patent literature cited in the description


- **SAKAI et al.** *Macromolecules,* 2008, vol. 41, 5379-5384
- **MATSUNAGA et al.** *Macromolecules,* 2009, vol. 42 (4), 1344-1351